# EUROPÄISCHE PATENTANMELDUNG

(11) **EP 4 722 672 A1**
(43) Veröffentlichungstag der Anmeldung: **08.04.2026**
(21) Anmeldenummer: 25205542.1
(22) Anmeldetag: 30.09.2025
(51) Int. Cl.: G01L 1/14, A01L 7/00, A61B 5/103, A01K 29/00, A61B 5/00

(54) **TRITTELEMENT ZUR ERFASSUNG DER BODENREAKTIONSKRAFTVERTEILUNG DES FUSSES EINES HUFTIERS ODER EINES MENSCHEN**

(30) Priorität: 01.10.2024 DE 102024209605
(71) Anmelder: ContiTech Deutschland GmbH, 30175 Hannover (DE)
(72) Erfinder: Dr. Reck, Siegfried, 30175 Hannover (DE)
(74) Vertreter: Preusser, Andrea

(57) **Zusammenfassung**

Die vorliegende Erfindung betrifft ein Trittelement (2) zur Erfassung der Bodenreaktionskraftverteilung über die Bodenkontaktfläche des Fußes eines Huftiers oder des Fußes eines Menschen bei dessen Auftreten auf den Boden, mit einer Mehrzahl von Feder-Elementen (4), welche im Gebrauch zwischen dem Boden und dem Huf oder Fuß angeordnet sind, wobei die Feder-Elemente (4) als flächige Kraftmesssensoren (4) ausgebildet sind, welche jeweils wenigstens eine Mikrowellen-Streifenleitung (18) mit einer individuellen Eigenfrequenz aufweisen, welche sich proportional zu einer einwirkenden Kraft (F) ändert.

## Beschreibung

Die vorliegende Erfindung betrifft ein Trittelement zur Erfassung der Bodenreaktionskraftverteilung über die Bodenkontaktfläche des Fußes eines Huftiers oder des Fußes eines Menschen bei dessen Auftreten auf den Boden.

Die Erfindung betrifft die bewegungsbegleitende Messung der Kraftverteilung in den Aufstandsflächen der Extremitäten zur (sport-)medizinischen Analyse.

Für Pferde und vergleichbare Tiere wurde ein System entwickelt, das als Hufschuh an den Hufen befestigt werden kann. Unterhalb der natürlichen Aufstandsfläche des Tieres befindet sich bei jedem Huf ein Trittelement mit mehreren folienartigen Kraftsensoren, deren elektrischer Widerstand sich proportional zu der Kraft ändert, die orthogonal auf das Trittelement wirkt. Elastomere Distanzstücke verteilen diese Aufstandskraft auf die einzelnen Kraftsensoren, siehe DE 10 2021 211 795 A1. Um die Kräfte synchron zu messen, werden die Sensoren mit separaten Signalverstärkern verdrahtet.

Nachteilig hieran ist, dass die Folien-Sensoren eine nichtlineare Kennlinie und Hysterese aufweisen können. Außerdem können die Folien-Sensoren Setzungseffekte und bzw. oder eine Temperaturabhängigkeit aufweisen. Aus diesen Gründen ist der Aufwand für die Auswertung der Kraftsignale erheblich.

Eine Aufgabe der vorliegenden Erfindung ist es, ein verbessertes Trittelement der eingangs beschriebenen Art bereit zu stellen. Insbesondere soll die Erfassung der einwirkenden Kräfte vereinfacht werden. Zumindest soll eine Alternative zu den bekannten Möglichkeiten geschaffen werden.

Die Aufgabe wird erfindungsgemäß durch eine Trittelement mit den Merkmalen gemäß Patentanspruch 1 gelöst. Vorteilhafte Weiterbildungen sind in den Unteransprüchen beschrieben.

Somit betrifft die vorliegende Erfindung ein Trittelement zur Erfassung der Bodenreaktionskraftverteilung über die Bodenkontaktfläche des Fußes eines Huftiers oder des Fußes eines Menschen bei dessen Auftreten auf den Boden, mit einer Mehrzahl von Feder-Elementen, welche im Gebrauch zwischen dem Boden und dem Huf oder Fuß angeordnet sind, wobei die Feder-Elemente als flächige Kraftmesssensoren ausgebildet sind, welche jeweils wenigstens eine Mikrowellen-Streifenleitung mit einer individuellen Eigenfrequenz aufweisen, welche sich proportional zu einer einwirkenden Kraft ändert.

Dies kann es ermöglichen, eine Krafterfassung, eine Kraftbestimmung bzw. eine Bestimmung einer Bodenreaktionskraftverteilung mittels einer Mikrowellen-Streifenleitung auf die zuvor beschriebenen Anwendungen anzuwenden und dessen Eigenschaften und Vorteile dort zu nutzen.

Grundsätzlich werden die Resonanzfrequenzen der Resonatoren durch ihre geometrischen Abmessungen definiert. In Anwendungen, bei denen der Bauraum limitiert ist, kann der Raum innerhalb der Feder-Elemente mit Abstimmung der Resonatoren über die Länge und die Dielektrizitätszahl der Füllung in der Nut genutzt werden.

Dies kann die Bestimmung der einwirkenden Kräfte mittels einer stetigen und differenzierbaren Kennlinie ermöglichen, da die Feder-Elemente im Bereich geringer Verformung betrieben werden können, weil die Resonanzfrequenz auch bei geringer Dehnung sehr empfindlich reagieren kann. Hierdurch kann auch eine Hysterese vermieden werden.

Dies kann eine geringere Temperaturabhängigkeit der Messwerte begünstigen, da als Werkstoff der Feder-Elemente mit Material mit einem kleineren Temperaturkoeffizienten als das Material der ohmschen Widerstände, vorzugsweise als die Widerstandspaste der gedruckten ohmschen Widerstände, verwendet werden kann.

Es kann eine vergleichsweise flexible Anwendung erfolgen, da der Messbereich für die Kraft durch die Gestaltung der Feder-Elemente an die jeweilige Anwendung angepasst werden kann.

Es kann auch die Verdrahtung geringgehalten werden, da für die erfindungsgemäße Schaltung lediglich zwei gemeinsame Leitungen für alle Feder-Elemente als Kraftsensoren notwendig sind, nämlich lediglich eine Speiseleitung und Erde, während N Widerstands-Kraftsensoren mindestens N+1 Leitungen erfordern (bei differentieller Auswertung sind es 2*N Leitungen).

Gemäß einem Aspekt der Erfindung weisen die Mikrowellen-Streifenleitungen der Feder-Elemente jeweils wenigstens auf:
- eine elektrisch isolierte Leiterfläche, welche sich flächig senkrecht zur Richtung der einwirkenden Kraft erstreckt,
- einen Mikrowellen-Resonator, welcher sich senkrecht zur Richtung der einwirkenden Kraft erstreckt und gegenüber dem elektrisch isolierten Leiterfläche entlang der Richtung der einwirkenden Kraft elektrisch isoliert ist, und
- eine Leiterbahn, welcher sich senkrecht zur Richtung der einwirkenden Kraft erstreckt, gegenüber dem elektrisch isolierten Leiterfläche entlang der Richtung der einwirkenden Kraft elektrisch isoliert und gegenüber dem Mikrowellen-Resonator in der Ebene senkrecht zur Richtung der einwirkenden Kraft parallel verlaufend und beabstandet angeordnet ist.

Dies kann eine konkrete Möglichkeit der Umsetzung darstellen.

Gemäß einem weiteren Aspekt der Erfindung erstreckt sich die elektrisch isolierte Leiterfläche in der Fläche senkrecht zur Richtung der einwirkenden Kraft über den Mikrowellen-Resonator und über die Leiterbahn hinaus.

Dies kann eine konkrete Möglichkeit der Umsetzung darstellen. Dies kann die abschirmende Wirkung der elektrisch isolierten Leiterfläche gegenüber dem Mikrowellen-Resonator und der Leiterbahn verbessern.

Gemäß einem weiteren Aspekt der Erfindung weist das Trittelement ferner eine Mess- und Auswerte-Elektronik auf, welche ausgebildet und eingerichtet ist, die aktuelle Eigenfrequenz jedes Feder-Elements zu erfassen und aus der erfassten Eigenfrequenz die jeweils einwirkende Kraft pro Feder-Element zu bestimmen.

Dies kann eine konkrete Möglichkeit der Umsetzung darstellen, um die Messwerte der Feder-Elemente als Kraftsensoren direkt auszuwerten.

Gemäß einem weiteren Aspekt der Erfindung weist das Trittelement ferner eine Antenne auf, welche ausgebildet, drahtlos mit außerhalb des Trittelements wenigstens unidirektional, vorzugsweise bidirektional, zu kommunizieren, wobei die Mess- und Auswerte-Elektronik ferner ausgebildet und eingerichtet ist, die bestimmten Kräfte pro Feder-Element über die Antenne nach außerhalb zu kommunizieren.

Dies kann eine konkrete Möglichkeit darstellen, die Messwerte nach außerhalb des Trittelements wie z.B. an ein mobiles Endgerät wie z.B. an ein Smartphone zur Verfügung zu stellen.

Gemäß einem weiteren Aspekt der Erfindung weist wenigstens ein Feder-Element, vorzugsweise weisen mehrere Feder-Elemente jeweils, besonders vorzugsweise weise alle Feder-Elemente jeweils, auf:
- ein Paar elektrisch isolierter Leiterflächen, welche sich flächig senkrecht zur Richtung der einwirkenden Kraft erstrecken und parallel zueinander verlaufen,
- ein Paar von Mikrowellen-Resonatoren, welcher sich senkrecht zur Richtung der einwirkenden Kraft erstrecken, jeweils gegenüber der entsprechenden elektrisch isolierten Leiterfläche entlang der Richtung der einwirkenden Kraft elektrisch isoliert sowie entlang der Richtung der einwirkenden Kraft zueinander elektrisch isoliert sind und parallel zueinander verlaufen, und
- ein Paar von Leiterbahnen, welche sich senkrecht zur Richtung der einwirkenden Kraft erstrecken, jeweils gegenüber der entsprechenden elektrisch isolierten Leiterfläche entlang der Richtung der einwirkenden Kraft elektrisch isoliert sowie entlang der Richtung der einwirkenden Kraft zueinander elektrisch isoliert, jeweils gegenüber dem entsprechenden Mikrowellen-Resonator in der Ebene senkrecht zur Richtung der einwirkenden Kraft parallel verlaufend und beabstandet angeordnet sind.

Dies kann die Verdopplung der Mikrostreifenleitung ermöglichen, um pro Feder-Element zwei Messwerte derselben einwirkenden Kraft zu erhalten, welche beide ausgewertet und vorzugsweise gemittelt werden können, um Messungenauigkeiten der einzelnen Feder-Elemente zu relativieren.

Gemäß einem weiteren Aspekt der Erfindung sind die beiden Mikrowellen-Resonatoren und bzw. oder die beiden Leiterbahnen entlang der Richtung der einwirkenden Kraft durch eine elastische Vergussmasse zueinander beabstandet und gegenüber einander elektrisch isoliert.

Dies kann einen sicheren Abschluss der Mikrowellen-Resonatoren und bzw. oder der Leiterbahnen gegenüber dem Umgebung und deren Einflüssen wie Schmutz, Feuchtigkeit und dergleichen ermöglichen, ohne die Funktionsweise der Feder-Elemente ungebührlich einzuschränken.

Gemäß einem weiteren Aspekt der Erfindung sind die Feder-Elemente elastisch ausgebildet. Dies kann die Kraftübertragung bzw. die Verteilung der einwirkenden Kräfte auf die Feder-Elemente bzw. deren Mikrostreifenleitungen verbessern.

Gemäß einem weiteren Aspekt der Erfindung sind die Feder-Elemente an derjenigen Oberfläche des Trittelements angeordnet, welche bei Gebrauch dem Boden zugewandt ist. Dies kann die Kraftübertragung der einwirkenden Kräfte auf die Feder-Elemente bzw. deren Mikrostreifenleitungen verbessern.

Gemäß einem weiteren Aspekt der Erfindung ist das Trittelement bogenförmig ausgebildet und entspricht der randseitigen Kontur des Hufes oder des Fußes, wobei die Feder-Elemente am Bogen des Trittelements angeordnet und zueinander beabstandet sind. Dies kann es begünstigen, die für die Erfassung der Bodenreaktionskraftverteilung des Fußes eines Huftiers oder eines Menschen relevanten Messdaten zu erfassen bzw. eine Bestimmung der Bodenreaktionskraftverteilung zu ermöglichen, welche repräsentativ ist.

Mehrere Ausführungsbeispiele und weitere Vorteile der Erfindung werden nachstehend im Zusammenhang mit den folgenden Figuren erläutert. Darin zeigt:
- Figur 1: eine schematische Darstellung eines erfindungsgemäßen Trittelements mit einer Mehrzahl von Feder-Elementen gemäß eines ersten Ausführungsbeispiels;
- Figur 2: einen Querschnitt A-A durch eines der Feder-Elemente der Figur 1;
- Figur 3: die Darstellung der Figur 2 unter Krafteinwirkung;
- Figur 4: eine Hochfrequenz-Schaltung des Trittelements der Figur 1;
- Figur 5: eine beispielshafte Messung der Resonanzfrequenzen der Feder-Elemente der Figur 1 unter Krafteinwirkung;
- Figur 6: einen Querschnitt A-A durch eines der Feder-Elemente gemäß eines zweiten Ausführungsbeispiels; und
- Figur 7: die Darstellung der Figur 6 unter Krafteinwirkung.

Die Beschreibung der o.g. Figuren erfolgt in kartesischen Koordinaten mit einer Längsachse X, einer zur Längsachse X senkrecht ausgerichteten Querachse Y sowie einer sowohl zur Längsachse X als auch zur Querachse Y senkrecht ausgerichteten vertikalen Achse Z, welche der Richtung der Schwerkraft entspricht. Die Längsachse X kann auch als Tiefe X, die Querachse Y auch als Breite Y und die vertikale Achse Z auch als Höhe Z bezeichnet werden. Die Längsachse X und die Querachse Y bilden gemeinsam die Horizontale X, Y, welche auch als horizontale Ebene X, Y bezeichnet werden kann. Die Längsachse X, die Querachse Y und die vertikale Achse Z können gemeinsam auch als Raumrichtungen X, Y, Z bzw. als kartesische Raumrichtungen X, Y, Z bezeichnet werden.

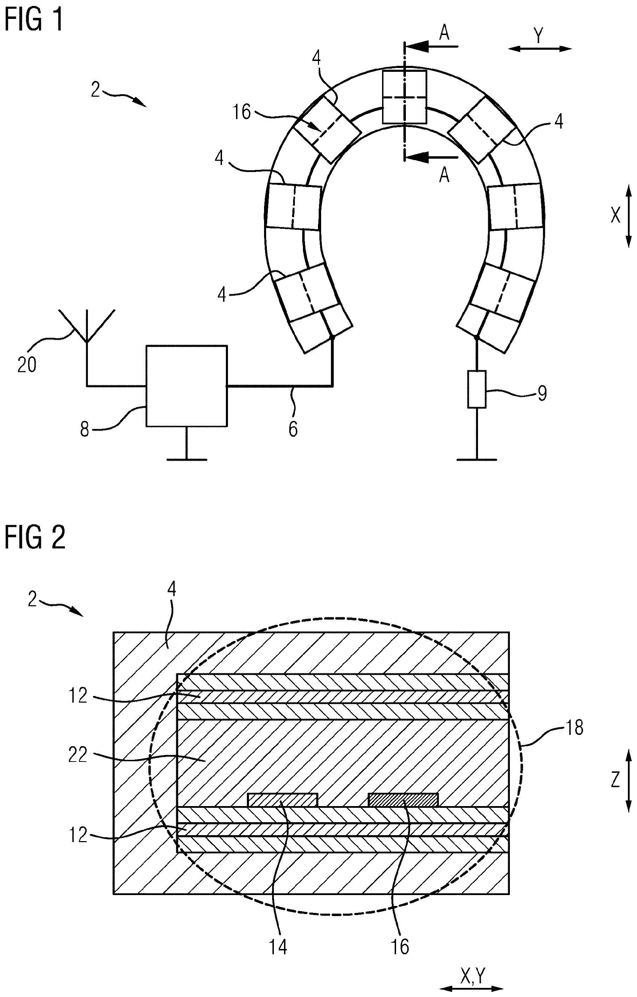

Die Figur 1 zeigt eine Übersicht des erfindungsgemäßen U-förmiges Trittelements 2 am Beispiel der Anwendung an einem Pferdehuf (nicht dargestellt). Das Trittelement 2 enthält mehrere Feder-Elemente 4, welches über eine Speiseleitung 6 elektrisch miteinander verbunden sind. Der Eingang der Speiseleitung 6 ist mit einer Mess- und Auswerte-Elektronik 8 und der Ausgang über einen 50-Ohm-Widerstand 9 mit dem Masse-Potenzial elektrisch leitfähig verbunden. Die Mess- und Auswerte-Elektronik 8 verfügt über einen internen (Festwert-)Speicher, eine geeignete Rechenleistung und eine Kommunikationseinrichtung (nicht dargestellt).

Die Figur 2 zeigt einen Querschnitt A-A durch eines der Feder-Elemente 4. Auf beiden Innenflächen jedes Feder-Elements 4 befindet sich jeweils eine beidseitig elektrisch isolierte Leiterfläche 12, die elektrisch mit dem Massepotenzial, vgl. Figur 1, elektrisch leitfähig verbunden ist. Auf der Isolierung einer der Leiterflächen 12 befindet sich eine Leiterbahn, die als Mikrowellen-Resonator 14 dient. Der Mikrowellen-Resonator 14 wird bei der Messung von einer parallel verlaufenden Leiterbahn 16 angeregt, welche Teil der gemeinsamen Speiseleitung 6 ist, die durch alle Feder-Elemente 4 hindurch verläuft. Die Leiterstrukturen 12, 14, 16 in den Federelementen 4 bilden jeweils eine Mikrowellen-Streifenleitung 18.

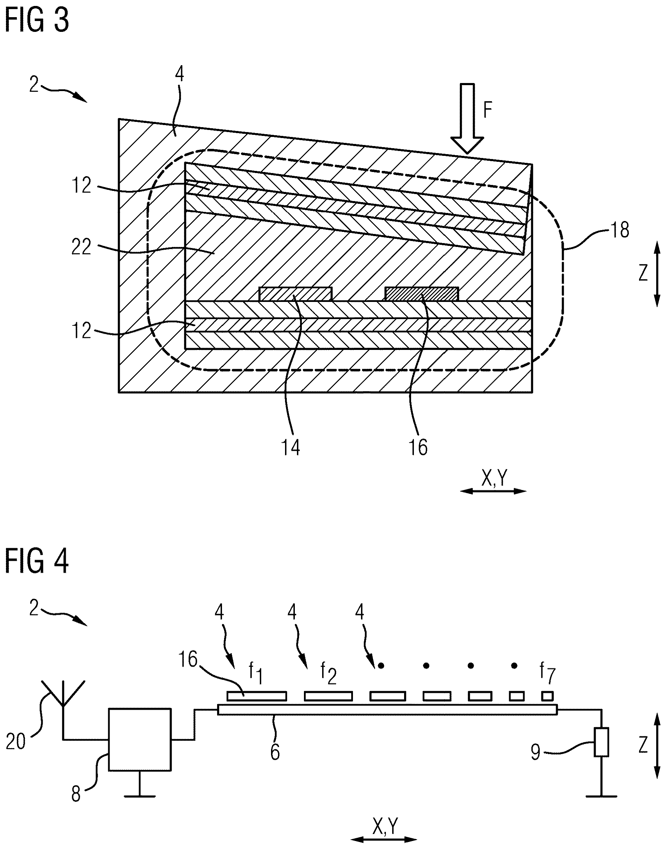

Die Resonanzfrequenzen der Mikrowellen-Streifenleitungen 18 sind konstruktiv so abgestimmt, dass die einzelnen Resonanzfrequenzen inkl. ihrer Oberwellen jeweils nur einmal in dem verwendeten Frequenzbereich vorkommen. Mit anderen Worten weist jedes Feder-Element 4 eine eigene Eigenfrequenz auf, welche bei dem Trittelement 2 nur einmal vorgesehen ist. Die Figur 4 zeigt die Hochfrequenz-Schaltung des Trittelements 2 (ohne die Leiterflächen 12).

Zum Schutz vor eindringenden Fremdstoffen wie z.B. Wasser, die das Frequenzverhalten der Mikrowellen-Streifenleitungen 18 verändern können, wird der Raum zwischen den Innenflächen der Feder-Elemente 4 mit einer elastischen Vergussmasse (nicht dargestellt) gefüllt, die so weich ist, dass ihr Einfluss auf die Kraftmessung vernachlässigt werden kann.

Zum Schutz vor eindringenden Fremdstoffen wie z.B. Wasser, die das Frequenzverhalten der Mikrowellen-Streifenleitungen 18 verändern, wird der Raum zwischen den Innenflächen der Feder-Elemente 4 mit einer elastischen Vergussmasse 22 gefüllt, die so weich ist, dass ihr Einfluss auf die Kraftmessung vernachlässigt werden kann.

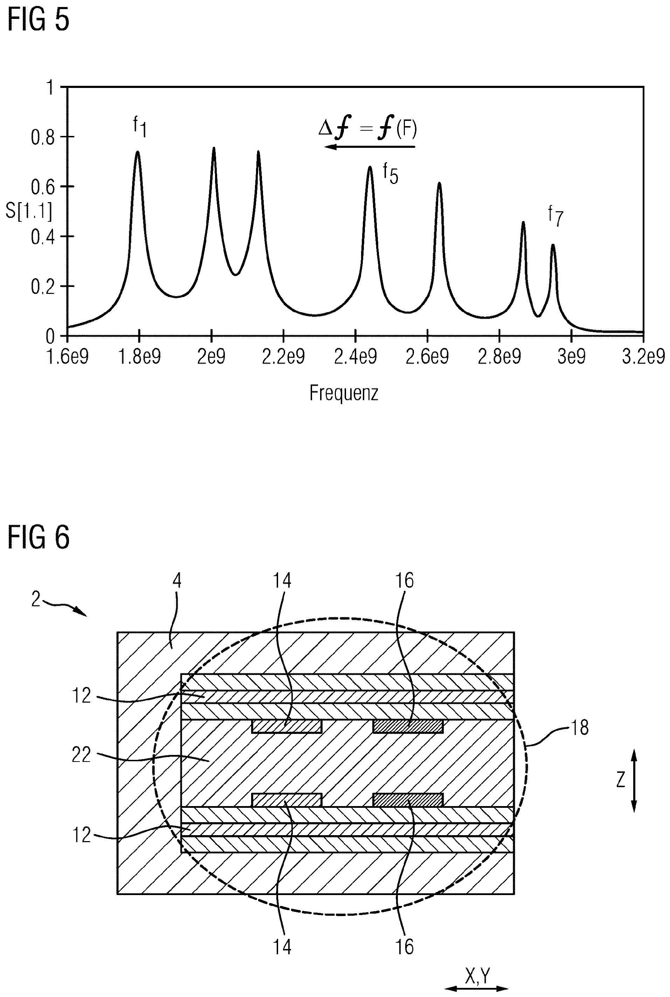

Die zu messende (Teil-)Kraft F verformt nun bei Gebrauch die Feder-Elemente 4, siehe Figur 3, und verstimmt dadurch dessen Mikrowellen-Streifenleitungen 18, so dass sich deren Resonanzfrequenz zu niedrigeren Werten verschiebt, vgl. Figur 5.

Im Anschluss an die Herstellung des Trittelements 2 wird für alle Feder-Elemente 4 der Zusammenhang zwischen der Kraft und der entsprechenden Resonanzfrequenz der Mikrowellen-Streifenleitungen 18 ermittelt. Die Daten werden im Festwertspeicher der Mess- und Auswerte-Elektronik 8 als Kalibrierdaten abgelegt.

Bei der Messung legt die Mess- und Auswerte-Elektronik 8 ein HochfrequenzSignal variabler Frequenz an die Speiseleitung 6 und misst dabei den Betrag ihres Reflexionsfaktors. Aus dem Betragsspektrum ermittelt die Mess- und Auswerte-Elektronik 8 die Werte der Resonanzfrequenzen der Mikrowellen-Streifenleitungen 18 in den einzelnen Feder-Elementen 4. Mit Hilfe der gespeicherten Kalibrierdaten berechnet die Mess- und Auswerte-Elektronik 8 daraus die Kräfte F, welche aktuell auf die einzelnen Feder-Elemente 4 wirken.

Die Ergebnisse der Auswertung überträgt die Mess- und Auswerte-Elektronik 8 über eine Antenne 20 drahtlos an ein externes System wie z. B. ein Smartphone (nicht dargestellt). Dort kann dann eine Nutzung der Kraftmessdaten des Trittelements 2 erfolgen.

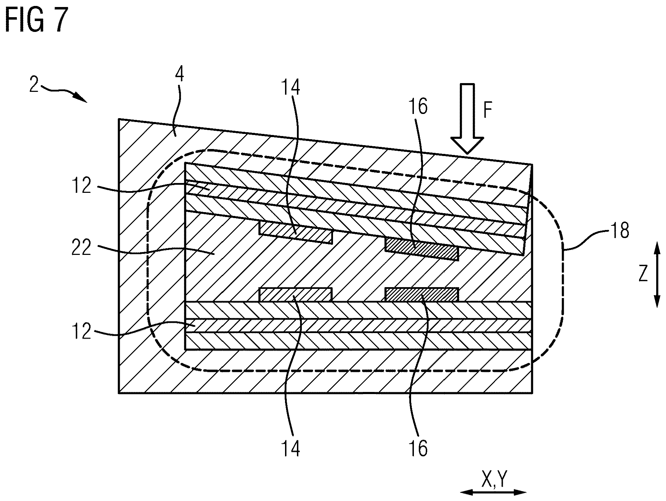

Die Figuren 6 und 7 zeigen ein zweites Ausführungsbeispiel der Erfindung, bei welchem pro Trittelement 2 bzw. pro Feder-Element 4 sowohl auf der unteren Fläche des Feder-Elements 4 als auch auf dessen oberer Fläche jeweils eine Mikrowellen-Streifenleitung 18 mit jeweils einer elektrisch isolierten Leiterfläche 12, einem Mikrowellen-Resonator 14 einer parallel verlaufenden Leiterbahn 16 angeordnet sind, um beide Messignale pro Feder-Element 4 seitens der Mess- und Auswerte-Elektronik 8 und hieraus, beispielsweise durch Mittelwertbildung, einem genauere Kraftmesswert pro Feder-Element 4 zu erhalten.

Mit der symmetrischen Variante der Anordnung der Figuren 6 und 7 können zusätzlich auch die Übertragungsparameter (S21 und S12) gemessen werden, um die Ergebnisse zu verfeinern oder um Redundanz zu schaffen. Diese Variante benötigt einen Richtkoppler als zusätzliche Komponente (nicht dargestellt).

### Bezugszeichenliste (Teil der Beschreibung)

- A-A: Querschnitt
- F: einwirkende Kraft
- f₁-f₇: erste bis siebte Eigenfrequenz der Mikrowellen-Streifenleitung 18

- X: Längsachse; Tiefe
- Y: Querachse; Breite
- Z: vertikale Achse; Höhe
- X, Y: Horizontalen; horizontale Ebene

- 2: (bogenförmiges, hufförmiges bzw. U-förmiges) Trittelement
- 4: Feder-Elemente; Kraftmesssensoren
- 6: Speiseleitung
- 8: Mess- und Auswerte-Elektronik
- 9: 50-Ohm-Widerstand
- 12: elektrisch isolierte Leiterfläche der Mikrowellen-Streifenleitung 18
- 14: Mikrowellen-Resonator der Mikrowellen-Streifenleitung 18
- 16: parallele Leiterbahn der Speiseleitung 6 bzw. der Mikrowellen-Streifenleitung 18
- 18: Mikrowellen-Streifenleitung
- 20: Antenne
- 22: elastische Vergussmasse

## Patentansprüche

1. Trittelement (2) zur Erfassung der Bodenreaktionskraftverteilung über die Bodenkontaktfläche des Fußes eines Huftiers oder des Fußes eines Menschen bei dessen Auftreten auf den Boden,
mit einer Mehrzahl von Feder-Elementen (4), welche im Gebrauch zwischen dem Boden und dem Huf oder Fuß angeordnet sind,
wobei die Feder-Elemente (4) als flächige Kraftmesssensoren (4) ausgebildet sind, welche jeweils wenigstens eine Mikrowellen-Streifenleitung (18) mit einer individuellen Eigenfrequenz aufweisen, welche sich proportional zu einer einwirkenden Kraft (F) ändert.

2. Trittelement (2) nach Anspruch 1,
wobei die Mikrowellen-Streifenleitungen (18) der Feder-Elemente (4) jeweils wenigstens aufweisen:
• eine elektrisch isolierte Leiterfläche (12), welche sich flächig senkrecht zur Richtung der einwirkenden Kraft (F) erstreckt,
• einen Mikrowellen-Resonator (14), welcher sich senkrecht zur Richtung der einwirkenden Kraft (F) erstreckt und gegenüber dem elektrisch isolierten Leiterfläche (12) entlang der Richtung der einwirkenden Kraft (F) elektrisch isoliert ist, und
• eine Leiterbahn (16), welcher sich senkrecht zur Richtung der einwirkenden Kraft (F) erstreckt, gegenüber dem elektrisch isolierten Leiterfläche (12) entlang der Richtung der einwirkenden Kraft (F) elektrisch isoliert und gegenüber dem Mikrowellen-Resonator (14) in der Ebene senkrecht zur Richtung der einwirkenden Kraft (F) parallel verlaufend und beabstandet angeordnet ist.

3. Trittelement (2) nach Anspruch 2,
wobei sich die elektrisch isolierte Leiterfläche (12) in der Fläche senkrecht zur Richtung der einwirkenden Kraft (F) über den Mikrowellen-Resonator (14) und über die Leiterbahn (16) hinaus erstreckt.

4. Trittelement (2) nach einem der vorangehenden Ansprüche,
ferner mit einer Mess- und Auswerte-Elektronik (8), welche ausgebildet und eingerichtet ist, die aktuelle Eigenfrequenz jedes Feder-Elements (4) zu erfassen und aus der erfassten Eigenfrequenz die jeweils einwirkende Kraft (F) pro Feder-Element (4) zu bestimmen.

5. Trittelement (2) nach Anspruch 4,
ferner mit einer Antenne (22), welche ausgebildet, drahtlos mit außerhalb des Trittelements (2) wenigstens unidirektional, vorzugsweise bidirektional, zu kommunizieren,
wobei die Mess- und Auswerte-Elektronik (8) ferner ausgebildet und eingerichtet ist, die bestimmten Kräfte (F) pro Feder-Element (4) über die Antenne (22) nach außerhalb zu kommunizieren.

6. Trittelement (2) nach einem der vorangehenden Ansprüche,
wobei wenigstens ein Feder-Element (4), vorzugsweise mehrere Feder-Elemente (4) jeweils, besonders vorzugsweise alle Feder-Elemente (4) jeweils, aufweist:
• ein Paar elektrisch isolierter Leiterflächen (12), welche sich flächig senkrecht zur Richtung der einwirkenden Kraft (F) erstrecken und parallel zueinander verlaufen,
• ein Paar von Mikrowellen-Resonatoren (14), welcher sich senkrecht zur Richtung der einwirkenden Kraft (F) erstrecken, jeweils gegenüber der entsprechenden elektrisch isolierten Leiterfläche (12) entlang der Richtung der einwirkenden Kraft (F) elektrisch isoliert sowie entlang der Richtung der einwirkenden Kraft (F) zueinander elektrisch isoliert sind und parallel zueinander verlaufen, und
• ein Paar von Leiterbahnen (16), welche sich senkrecht zur Richtung der einwirkenden Kraft (F) erstrecken, jeweils gegenüber der entsprechenden elektrisch isolierten Leiterfläche (12) entlang der Richtung der einwirkenden Kraft (F) elektrisch isoliert sowie entlang der Richtung der einwirkenden Kraft (F) zueinander elektrisch isoliert, jeweils gegenüber dem entsprechenden Mikrowellen-Resonator (14) in der Ebene senkrecht zur Richtung der einwirkenden Kraft (F) parallel verlaufend und beabstandet angeordnet sind.

7. Trittelement (2) nach Anspruch 6,
wobei die beiden Mikrowellen-Resonatoren (14) und/oder die beiden Leiterbahnen (16) entlang der Richtung der einwirkenden Kraft (F) durch eine elastische Vergussmasse (22) zueinander beabstandet und gegenüber einander elektrisch isoliert sind.

8. Trittelement (2) nach einem der vorangehenden Ansprüche,
wobei die Feder-Elemente (4) elastisch ausgebildet sind.

9. Trittelement (2) nach einem der vorangehenden Ansprüche,
wobei die Feder-Elemente (4) an derjenigen Oberfläche des Trittelements (2) angeordnet sind, welche bei Gebrauch dem Boden zugewandt ist.

10. Trittelement (2) nach einem der vorangehenden Ansprüche,
wobei das Trittelement (2) bogenförmig ausgebildet ist und der randseitigen Kontur des Hufes oder des Fußes entspricht,
wobei die Feder-Elemente (4) am Bogen des Trittelements (2) angeordnet und zueinander beabstandet sind.
